# EUROPEAN PATENT SPECIFICATION

(11) **EP 4 480 519 B1**
(45) Date of publication and mention of the grant of the patent: **18.02.2026**
(21) Application number: 23461608.4
(22) Date of filing: 18.06.2023
(51) Int. Cl.: A61M 11/00, A61M 16/00, A61M 15/00, A61M 16/08, A61M 11/06, A61M 16/10, A61M 16/06

(54) **NEBULISATION HEAD ATTACHMENT FOR TEMPERATURE STABILISATION**
VERNEBELUNGSKOPFBEFESTIGUNG ZUR TEMPERATURSTABILISIERUNG
FIXATION DE TÊTE DE NÉBULISATION POUR LA STABILISATION DE LA TEMPÉRATURE

(43) Date of publication of application: 25.12.2024
(73) Proprietor: Uniwersytet Zielonogórski, 65-417 Zielona Góra (PL)
(72) Inventor: Doligalski, Michal, 65-395 Zielona Gora (PL); Mrugalski, Marcin, 65-128 Zielona Gora (PL); Kasperkowiak, Anna, 63-230 Witaszyce (PL); Ochowiak, Marek, 62-025 Kostrzyn (PL)
(74) Representative: JD&P Patent Attorneys Joanna Dargiewicz & Partners

(56) References cited:
- WO-A1-2017/174807
- WO-A1-2017/199215
- WO-A1-2018/222912

## Description

The object of the present invention is a nebulisation head attachment for temperature stabilisation.

Aerosol therapy involves the delivery of medication (active substance) by inhalation into the patient's airways in the form of a mist, with distilled water usually acting as the carrier. The therapy is usually carried out at home, without the supervision of medical staff, by unqualified persons. In particular, nebulisation therapy in children is problematic. For it to be effective, two conditions must be met: the patient should take deep breaths at a low frequency, the temperature of the mist should be close to the patient's body temperature (a low temperature may cause bronchospasm). Existing inhaler solutions including inhalers do not provide the possibility to monitor and control the nebulisation process. No control or control of the temperature of the aerosol is realised and therefore there is no control over the process itself. The lack of temperature stabilisation causes the process to be disrupted by external disturbances, mainly fluctuations in ambient temperature, device temperature, patient inhalation rate. The main disturbance to the process is the drop in aerosol temperature when the gas is expanded in the nebulisation (pneumatic) head.

In the nebulisation process, it is essential that the active ingredient is delivered correctly; a drop in the temperature of the expanding aerosol increases the mean droplet diameter and has a direct adverse effect on the patient's respiratory system. The aim should be to stabilise the temperature and thus ensure the correct droplet diameter. The droplet diameter can be determined at the experimental stage.

Placing a heater in the nebulisation vessel may cause degradation of the active substance. Direct heating of the liquid in the nebulisation vessel should be avoided.

### Prior art

US patent application US4564748A discloses an arrangement for controlling the operation of an inhalation therapy heater using a temperature sensor of the flowing medium so as to maintain a preset temperature level to which the flowing medium is heated during the warm-up period. Heater operation is periodically interrupted under the control of the heater temperature sensor during the warm-up period to minimise heating caused by over-temperature. The low temperature detector, which is switched off during the warm-up period, generates an alarm signal if the medium temperature remains below the lower limit at the end of the warm-up period.

Polish patent application P.395401 discloses a method and a device for controlling and monitoring an aerosol generation device for administering a dose of a substance by inhalation. The invention relates to a method for controlling and monitoring an aerosol generation device for administering a dose of a drug by inhalation by means of an aerosol generation control and dose monitoring device, wherein any type of supervised aerosol generation device for administering a dose of a drug by inhalation is used, preferably a metered dose nebulizer, an ultrasonic inhaler or any other similar type of inhaler with known performance characteristics, whereby during aerosol generation for dose delivery by means of the supervised device, a reading is made of the exceedance of a threshold dose of pressure in the pressure tubing connected to the mouthpiece of the supervised device, which signifies the start of the inhalation phase and the generation of aerosol by the supervised inhalation device is initiated until the threshold pressure in the pressure tubing is again exceeded, which is read as an exhalation phase or a pause in breathing, characterised by the following, that the device to be supervised is combined in an assembly with a device for controlling the generation of the aerosol and monitoring the dose to be dosed, which assembly is controlled according to the respiratory phases of inspiration and expiration, whereby an individual calibration of the device is carried out for each assembly thus formed, individually determining for it the threshold pressure value at the pressure sensor (4), recognised as the start of the inhalation phase and the exhalation phase, as well as the limit value for the volume of aerosol and the limit value for the duration of aerosol generation, after which aerosol generation is initiated at the dosage rate set, during which the individual measurements are recorded and summed in real time by the control and monitoring device, the individual measured instantaneous aerosol volumes used for aerosol dosing in each individual measuring cycle are recorded and summed up, as well as the instantaneous aerosol generation times measured in the individual measuring cycles are recorded and summed up and the feed rate is controlled and monitored, whereby the aerosol generation of the dose metering in the supervised inhalation device is terminated when the volume of aerosol generation used for dose metering, being the sum of the instantaneous volumes measured over the individual measurement cycles, exceeds the aerosol volume limit described as the aerosol volume, in which a dose equal to the preset dose is contained and/or when the time effectively used for aerosol generation in dose dispensing, being the sum of the instantaneous aerosol generation times measured in single measurement cycles, exceeds the limit value of the duration of aerosol generation, defined by the total time required to disperse the preset dose. The invention also relates to a device for controlling and monitoring an aerosol generation device for dispensing a drug dose.

Polish patent application P.441932 relates to a nebuliser having a vertically arranged cylindrical nebulisation chamber (1) with an air inlet (1.1) with a filter in the lower base and an aerosol outlet (1.2) in the upper base. It is characterised by having at least one spigot (2.1, 2.2) in the lower base of the nebulisation chamber (1) for supplying liquid, each of which is connected by an inlet via a separate pump (3.1, 3.2), to a separate container (4.1, 4.2). Each spigot (2.1, 2.2) is connected by an outlet to a separate tube (5.1, 5.2), which has a decreasing cross-sectional area from the inlet and a helix-shaped axis line on the cone. In the nebulisation chamber (1) there is a fan (6).

Polish patent application P.429021 discloses an aerosol spraying device in which the working medium (liquid) to be treated is sprayed by a nebulizer (4), equipped with a spray cycle controller, comprising a supply tank (1) with an outlet opening (2) located below 1/3 of the height of said tank and connected to a working tank (3), in which the nebuliser (4) is located, and adjacent to the working vessel (3) is a watertight supply and control chamber (5) containing the power source (7) and the cycle control (6) of the device, which are connected to the nebuliser (4) by electrical wires.

Polish patent application P.441933 discloses a nebuliser with adjustable dispersed particle size having a vertically arranged cylindrical nebulisation chamber (1) with an aerosol outlet (1.1) in the upper base. It is characterised in that in the lower base of the nebulisation chamber (1) there is a first spigot (2) with a valve (3) for supplying gas and a second spigot (4) for supplying fluid, which inlet is connected via an injector (5) to a first fluid pump (6). A second fluid inlet (7) is connected to the injector (5). The second fluid inlet (4) is connected to a pipe (8), which has a decreasing cross-sectional area from the inlet and a helix-shaped axis line on the cone. In the nebulisation chamber (1) there is a fan (9), while downstream of the aerosol outlet (1.1) there is an aerosol particle size sensor (10) which communicates with a controller (11) which communicates with the first fluid pump (6), the fan (9) and the valve (3).

Polish patent application P.417567 describes a kit for the combined study of the properties of fibrous and biological aerosols, characterised in that it comprises a chamber proper (11) containing an opening window (17) of the chamber, an agitator (12) with variable speed, an optical fibre meter (14) an optical particle meter (15), an aspirator (13) with a pump (16) and a filter (1) between them, valve (2) and rotameter (3), an air outlet from the proper chamber (11) with a filter (1) and with an electrostatic particle neutraliser (7) and an air filter (1) mounted in the air inlet, between which are mounted in parallel a nebuliser (4), a tube generator (5) and a humidifier (6), each with separately mounted rotameters (3) and valves (2). A method of testing the properties of fibrous and biological aerosols is also an object of the invention. A method for combined testing of aerosol properties is also an object of the application.

PCT application published as WO2018/222912 A1 discloses devices, systems, and methods which permit ventilation therapy concurrent with humidity and aerosol drug delivery. Exemplary mixer-heaters employ alternating actuation of humidity and drug nebulizers and may use a single constant power setting for the heating section while keeping a controlled outlet temperature over the course of treatment.

PCT application published as WO2017/199215 A1 discloses a medication delivery system having a holding chamber capable of delivering dosages of medicament from a metered dose inhaler. The holding chamber includes an actuator detector, flow detector and display. In another embodiment, a medication delivery system includes a holding chamber having an input and an output end, a metered dose inhaler operably coupled to the input end of the holding chamber, and a metered dose inhaler identifier associated with the holding chamber and operable to identify the metered dose inhaler coupled to the holding chamber.

Finally, PCT application published as WO2017/174807 A1 discloses a device for monitoring adherence of a patient to a prescribed regimen is provided. The device has a pulmonary delivery device fluid, an adapter in communication with the delivery device and a dispenser in fluid communication with the adapter. A pressurized drug, contained in the pulmonary delivery device, can be delivered from the pulmonary delivery device to the patient through the adapter and the dispenser. A sensor is mounted to the adapter or the dispenser for detecting a temperature or pressure change caused by the movement of the drug from the pulmonary delivery device to the patient and generating a feedback signal based on the change. The feedback signal is further processed for generating a signal indicating a successful deliver of the drug to the patient.

There are documents in the state of the art indicating a correlation between the temperature of the aerosol used in the nebulisation process and the particle size produced in the process, which consequently affects the deposition of the active substance in the patient's airways, but no solution for temperature stabilisation in the form of a nebulisation head attachment for temperature stabilisation has been disclosed. There is currently a lack of ways (devices, solutions) to stabilise the temperature in the nebulisation process and thus ensure the correct size of the aerosol droplets used in the nebulisation process.

The aim of the invention was to solve the problem of delivering aerosol droplets of a certain size during the nebulisation process, which are important for the application of this solution in medicine and, in particular, in aerosol therapy.

This was achieved by developing a nebulisation head attachment for temperature stabilisation.

Thus, the object of the present invention is a nebulization head attachment for temperature stabilization according to independent claim 1. Preferred embodiments are disclosed in the dependent claims.

Examples of the realisation of the nebulisation head attachment for temperature stabilisation according to the present invention are shown in the figures of the figure, where:

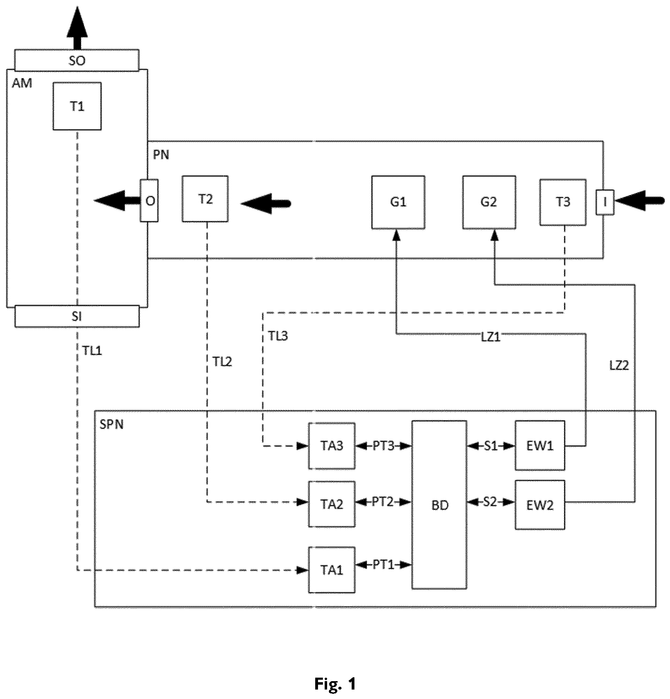

Fig. 1 shows a diagram of the nebulisation head attachment according to the present disclosure,

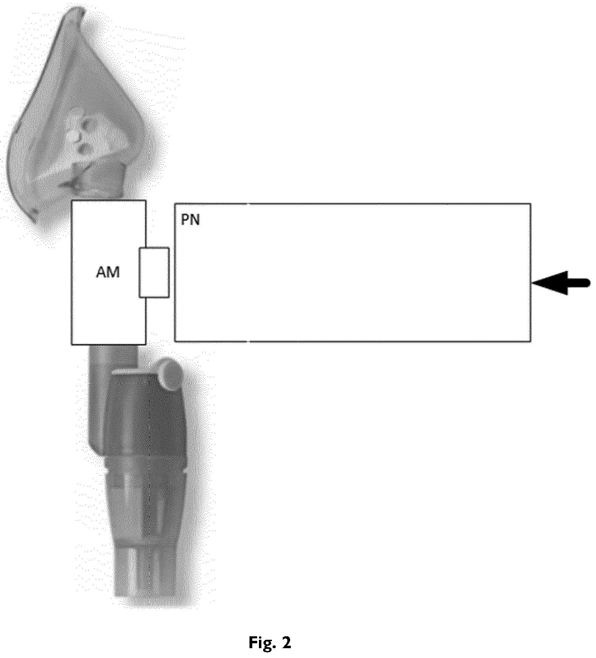

Fig. 2 shows the connection of the nebulisation head attachment to the nebulisation head and mask.

In one realisation example, the nebulisation head attachment for temperature stabilisation in aerosol therapies involving the delivery of medication by inhalation into the patient's airways in the form of a mist according to the present disclosure comprises the following components:
- a control adapter (PN) in the form of a sealed container fitted with
   ∘ an air intake opening (I) of any shape,
   ∘ an air outlet opening (O) of any shape, in particular circular, allowing direct or indirect connection to a nebuliser air inlet,
- a mixing adapter (AM) containing three holes made in the form of a solid, in particular in the form of a sleeve, which is connected by:
   ∘ the air outlet opening (O) to the control adapter (PN)
   ∘ an opening (SI) connecting to a nebulisation head, made in particular in the form of a circle and adapted to the standard diameters of the nebulisation heads, for connection to any nebulisation head, whereby the dimension and shape of the opening may be adapted to that of the air inlet of the nebulisation head,
   ∘ an opening (SO) to an application element in the form of a mask, nasal assembly, spray nozzle

   - the control and measurement system (SPN) of the control adapter (SN) equipped with:
      ∘ a first temperature analysis block (TA1) connected via a first data line TL1 to a first temperature sensor (T1) located in the mixing adapter (AM), which analyses the final mist temperature read from the first sensor (T1) against the target and critical temperature set by the decision block (BD) via a data line PT1
      ∘ a second temperature analysis block (TA2) connected via a second data line TL2 to a second temperature sensor (T2) located upstream of the air outlet (O) from the control adapter (PN) analysing the difference between the inlet temperature read by a third temperature sensor (T3) and the temperature value at the outlet of the control adapter (PN) read by a second temperature sensor (T2)
      ∘ a third temperature analysis block (TA3) connected via a third data line TL3 to the third temperature sensor (T3), analysing the system input temperature and the output temperature (T1);
      ∘ wherein the control and measurement system (SPN) comprises temperature analysis blocks (TA1, TA2, TA3), an actuators assemblies (EW1, EW2), heating elements (G1, G2), and a decision-making block (BD), in particular made using microprocessor technology.

The temperature analysis blocks (TA1, TA2, TA3) transmit data via data lines PT1, PT2 and PT3 to the decision block (BD), which then communicates with the actuators (EW1, EW2) via data lines S1 and S2.

The actuators (EW1, EW2) are responsible for distributing the thermal power to the heating elements (G1 and G2) by applying the supply voltage to the heating elements (via the LZ1 and LZ2 data lines), in particular for controlling the power in a two-position control system or controlling the power using pulse width modulation (PWM).

In the control adapter (PN), air movement takes place from the inlet opening (I) to the outlet opening (O), whereby the air, after passing through the inlet opening (I), moves around the third temperature sensor (T3) and then successively around the heating elements (G2) and (G1), in such a way that it is heated by the heating elements made in the form of spirals (in particular spirals wrapped around ceramic moulds). The heated air then flows around the second temperature sensor (T2) and enters the mixing adapter (AM) via the outlet opening (O).

The outlet opening (O) is typically circular in shape and connects the control adapter (PN) to the mixing adapter (AM).

The mixing adapter (AM) contains a first temperature sensor (T1) in the outlet section, just before the opening (O), which measures the temperature of the cloud formed in the nebulisation vessel, mixed with the air heated in the control adapter (PN).

The heating element assembly (G2 and G1) comprises heaters, in particular of different wattages, placed in series or in parallel in the air flow entering through the control adapter (PN) and flowing out of the control adapter (PN) into the mixing adapter (AM).

The device (head attachment) can be coupled to any type of nebulisation head by connecting the air outlet element of the control adapter (PN) to the air input (inlet) of the nebulisation head via the mixing adapter (AM).

On the basis of temperature values from sensors T1 - aerosol (sensor located at the outlet of the mixing adapter (AM)), T2 - air temperature at the outlet of the heating unit, T3 - temperature at the inlet of the heating unit, more precisely on the basis of temperature difference T1 - T2 and additionally temperature T3, the nebulisation head attachment according to the present disclosure stabilises temperature T1 by selective switching of heating elements, thus increasing the temperature of air supplied to the nebulisation vessel. By stabilising the temperature within the set range, it is possible to produce an aerosol with the set parameters of average droplet diameters and to increase the temperature of the inhaled aerosol to values which minimise the risk of bronchospasm.

The control system selectively switches on the heating elements (G1, G1) when an output temperature T1 lower than the setpoint temperature is detected. The use of two heating elements with different wattages allows the air to be heated in a range of 3 power levels. In combination with the inlet temperature data of the heating unit (T3), it is possible to adapt the heating power to the temperature difference between the unit's inlet and outlet. In addition, it is possible to control the temperature (T1 and T2) within a range of critical values which, if exceeded, could result in injury (burns) to the patient's respiratory system. In a critical situation, it is possible to disconnect the heating unit in an emergency and to signal the occurrence of an emergency situation using the signal block.

Unlike existing solutions, in which the absence of heating elements results in a lowering of the temperature during the expansion process (isentropic process) of the gas inhaled by the patient, this solution enables effective temperature stabilisation.

## Claims

1. A nebulisation head attachment for temperature stabilisation in aerosol therapies involving the delivery of medication by inhalation into the patient's airways in the form of a mist, comprising:
• a control adapter (PN) in the form of a sealed container equipped with a
∘ an air intake opening (I) of any shape,
∘ an air outlet opening (O) of any shape, preferably circular, allowing direct or indirect connection to a nebuliser air inlet,
∘ at least two heating elements (G1) and (G2),
• a mixing adapter (AM) containing three holes made in the form of a solid, preferably in the form of a sleeve, which is connected by:
∘ air outlet opening (O) to the control adapter (PN),
∘ an opening (SI) connecting to a nebulisation head, preferably in the form of a circle, for connection to any nebulisation head,
∘ an opening (SO) connecting to an application element, preferably in the form of a mask, a nasal assembly, a spray nozzle,
• a control and measurement system (SPN) of the control adapter (SN) equipped with:
∘ a first temperature analysis block (TA1) connected via a first data line TL1 to a first temperature sensor (T1) located in the mixing adapter (AM), which analyses the final mist temperature read from the first sensor (T1) against the target and critical temperature set by the decision block (BD) via a first data line PT1,
∘ a second temperature analysis block (TA2) connected via a second data line TL2 to a second temperature sensor (T2) located upstream of air outlet (O) from the control adapter (PN) analysing the difference between the inlet temperature read by a third temperature sensor (T3) and the temperature value at the outlet of the control adapter (PN) read by the second temperature sensor (T2),
∘ a third temperature analysis block (TA3) connected via a third data line TL3 to the third temperature sensor (T3), analysing the system input temperature and the first (output) temperature (T1);
wherein the control and measurement system (SPN) further comprises actuators assemblies (EW1, EW2) and a decision block (BD), preferably made using microprocessor technology;
the temperature analysis blocks (TA1, TA2, TA3) transmit data via data lines PT1, PT2 and PT3 to the decision-making block (BD), which then communicates with the actuators (EW1, EW2) via data lines S1 and S2;
the actuators (EW1) and (EW2) are responsible for distributing the thermal power to the heating elements (G1) and (G2) by applying the supply voltage to the heating elements via the data lines (LZ1) and (LZ2).

2. The nebulisation head attachment according to claim 1, **characterised in that** the heating elements are in the form of spirals, preferably spirals wrapped around ceramic moulds.

3. The nebulisation head attachment according to claims 1 or 2, **characterised in that** the heating elements are heaters of different powers placed in series or in parallel. 1 or 2, **characterised in that** the heating elements are heaters of different power, placed in series or in parallel.

## Patentansprüche

1. Vernebelungskopfaufsatz zur Temperaturstabilisierung bei Aerosoltherapien, bei denen Medikamente in Form eines Nebels durch Inhalation in die Atemwege des Patienten abgegeben werden, enthaltend:
• einen Steueradapter (PN) in Form eines versiegelten Behälters, mit
∘ einer Lufteinlassöffnung (I) beliebiger Form,
∘ einer Luftauslassöffnung (O) beliebiger Form, vorzugsweise kreisförmig, die einen direkten oder indirekten Anschluss an den Lufteinlass des Verneblers ermöglicht,
∘ mindestens zwei Heizelementen (G1) und (G2),
• einen Mischadapter (AM) mit drei Löchern in Form eines Festkörpers,
vorzugsweise in Form einer Hülse, der verbunden ist durch:
∘ die Luftauslassöffnung (O) mit dem Steueradapter (PN),
∘ eine Öffnung (SI), die mit dem Vernebelungskopf verbunden ist, vorzugsweise in Form eines Kreises, zum Anschluss an einen beliebigen Vernebelungskopf,
∘ eine Öffnung (SO), die mit dem Applikationselement verbindet,
vorzugsweise in Form einer Maske, einer Nasenvorrichtung, einer Sprühdüse,
• ein Steuerungs- und Messsystem (SPN) des Steueradapters (SN), das aufweist:
∘ einen ersten Temperaturanalyseblock (TA1), der über eine erste Datenleitung TL1 mit einem ersten Temperatursensor (T1), der sich in dem Mischadapter (AM) befindet, verbunden ist und die vom ersten Sensor (T1) gemessene finale Temperatur des Nebels mit der von dem Entscheidungsblock (BD) über eine erste Datenleitung PT1 festgelegte Soll- und kritischen Temperatur vergleicht,
∘ einen zweiten Temperaturanalyseblock (TA2), der über eine zweite Datenleitung TL2 mit einem zweiten Temperatursensor (T2) verbunden ist, der sich stromaufwärts des Luftauslasses (O) des Steueradapters (PN) befindet und die Differenz zwischen der von einem dritten Temperatursensor (T3) gemessenen Einlasstemperatur und dem von dem zweiten Temperatursensor (T2) gemessenen Temperaturwert am Auslass des Steueradapters (PN) analysiert,
∘ einen dritten Temperaturanalyseblock (TA3), der über eine dritte Datenleitung TL3 mit dem dritten Temperatursensor (T3) verbunden ist und die System-Eingangstemperatur und die erste (Ausgangs-)Temperatur (T1) analysiert;
wobei das Steuerungs- und Messsystem (SPN) ferner Aktuatoren-Baugruppen (EW1, EW2) und einen Entscheidungsblock (BD) enthält, die vorzugsweise unter Verwendung von Mikroprozessortechnologie hergestellt sind; und die Temperaturanalyseblöcke (TA1, TA2, TA3) Daten über die Datenleitungen PT1, PT2 und PT3 an den Entscheidungsblock (BD) übertragen, der dann über die Datenleitungen S1 und S2 mit den Aktuatoren (EW1, EW2) kommuniziert;
wobei die Aktuatoren (EW1) und (EW2) für die Verteilung der thermischen Leistung auf die Heizelemente (G1) und (G2) zuständig sind, indem die Versorgungsspannung über die Datenleitungen (LZ1) und (LZ2) an die Heizelemente angelegt wird.

2. Vernebelungskopfaufsatz nach Anspruch 1, **dadurch gekennzeichnet, dass** die Heizelemente die Form von Spiralen haben, vorzugsweise Spiralen, die um Keramikformen gewickelt sind.

3. Der Vernebelungskopfaufsatz gemäß Anspruch 1 oder 2, **dadurch gekennzeichnet, dass** die Heizelemente Heizgeräte unterschiedlicher Leistung sind, die in Reihe oder parallel geschaltet sind.

## Revendications

1. Accessoire de tête de nébulisation pour une stabilisation de température dans des aérosolthérapies impliquant la fourniture d'un médicament par inhalation jusque dans les voies respiratoires d'un patient sous la forme d'un brouillard, comprenant :
un adaptateur de commande (PN) sous la forme d'un contenant hermétique équipé avec
- une ouverture d'admission d'air (I) de toute forme,
- une ouverture d'évacuation d'air (O) de toute forme, de préférence circulaire, permettant une liaison directe ou indirecte avec une admission d'air de nébuliseur,
- au moins deux éléments de chauffe (G1) et (G2),
un adaptateur de mélange (AM) contenant trois orifices réalisés sous la forme d'un solide, de préférence sous la forme d'un manchon, qui est relié par :
- une ouverture d'évacuation d'air (O) vers l'adaptateur de commande (PN),
- une ouverture (SI) reliant à une tête de nébulisation, de préférence sous la forme d'un cercle, pour une liaison avec toute tête de nébulisation,
- une ouverture (SO) reliant à un élément d'application, de préférence sous la forme d'un masque, d'un ensemble nasal, d'une buse de pulvérisation,
un système de commande et de mesure (SPN) de l'adaptateur de commande (PN) équipé avec :
- un premier bloc d'analyse de température (TA1) relié via une première ligne de données TL1 à un premier capteur de température (T1) situé dans l'adaptateur de mélange (AM), lequel analyse la température de brouillard finale lue depuis le premier capteur (T1) par rapport à la température cible et critique réglée par le bloc de décision (BD) via une première ligne de données PT1,
- un deuxième bloc d'analyse de température (TA2) relié via une deuxième ligne de données TL2 à un deuxième capteur de température (T2) situé en amont d'une évacuation d'air (0) depuis l'adaptateur de commande (PN), analysant la différence entre la température d'admission lue par un troisième capteur de température (T3) et la valeur de température au niveau de l'évacuation de l'adaptateur de commande (PN) lue par le deuxième capteur de température (T2),
- un troisième bloc d'analyse de température (TA3) relié via une troisième ligne de données TL3 au troisième capteur de température (T3), analysant la température d'entrée de système et la première température (sortie) (T1) ;
dans lequel le système de commande et de mesure (SPN) comprend en outre des assemblages d'actionneurs (EW1, EW2) et un bloc de décision (BD), de préférence réalisés à l'aide d'une technologie de microprocesseur ;
les blocs d'analyse de température (TA1, TA2, TA3) transmettent des données via des lignes de données PT1, PT2 et PT3 au bloc preneur de décision (BD), lequel communique alors avec les actionneurs (EW1, EW2) via des lignes de données S1 et S2 ;
les actionneurs (EW1) et (EW2) sont chargés de distribuer la puissance thermique aux éléments de chauffe (G1) et (G2) en appliquant la tension d'alimentation aux éléments de chauffe via les lignes de données (LZ1) et (LZ2).

2. Accessoire de tête de nébulisation selon la revendication 1, **caractérisé en ce que** les éléments de chauffe sont sous la forme de spirales, de préférence de spirales enroulées autour de moules en céramique.

3. Accessoire de tête de nébulisation selon la revendication 1 ou 2, **caractérisé en ce que** les éléments de chauffe sont des chauffages de puissances différentes, placés en série ou en parallèle.
